# EUROPÄISCHE PATENTSCHRIFT

(11) **EP 0 882 735 B1**
(45) Veröffentlichungstag und Bekanntmachung des Hinweises auf die Patenterteilung: **19.04.2000**
(21) Anmeldenummer: 98108657.2
(22) Anmeldetag: 13.05.1998
(51) Int. Cl.: C07J 1/00, C07J 15/00, C07J 63/00, C07J 53/00

(54) **9 Alpha-Hydroxy-8 Alpha-estra-1,3,5(10)-triene und Verfahren zu deren Herstellung**
9 Alpha-hydroxy-8 alpha-estra-1,3,5(10)-trienes and a process for their preparation
9 Alpha-hydroxy-8 alpha-estra-1,3,5(10)-triènes et un procédé de leur production

(30) Priorität: 04.06.1997 DE 19723390
(43) Veröffentlichungstag der Anmeldung: 09.12.1998
(73) Patentinhaber: Jenapharm GmbH & Co. KG, 07745 Jena (DE)
(72) Erfinder: Kosemund, Dirk, Dr., 99091 Erfurt (DE); Schwarz, Sigfrid, Prof. Dr., 07743 Jena (DE)

(56) Entgegenhaltungen:
- DE-A- 2 523 179
- US-A- 3 519 714
- BOVICELLI P ET AL: "Oxidation of natural targets by dioxiranes. Oxyfunctionalization of steroids" JOURNAL OF ORGANIC CHEMISTRY., Bd. 57, Nr. 7, 27. März 1992, Seiten 2182-2184, XP002077346 EASTON US
- M. MAUMY ET AL: "No 347. - Oxydation des Stéroïdes. III. - Photo-oxygénation sensibilisée de l'hydroxy-17.béta.- estradiène-4(5),9(10) one-3. Préparation de séco 9-10 stéroïdes et recyclisation en de nouveaux 19-nor stéroïdes" BULLETIN DE LA SOCIETE CHIMIQUE DE FRANCE. 2 PARTIE - CHIMIE ORGANIQUE, BIOCHIMIE., Nr. 7-8, Juli 1975 - August 1975, Seiten 1879-1882, XP002077347 PARIS FR
- CHEMICAL ABSTRACTS, vol. 104, no. 7, 17. Februar 1986 Columbus, Ohio, US; abstract no. 051013, LI J ET AL: "Synthesis of 11-substituted estriols" XP002077348 & YAOXUE XUEBAO (YHHPAL,05134870);85; VOL.20 (3); PP.181-7, Beijing Med. Coll.;Dep. Pharm. Chem.; Beijing; Peop. Rep. China (CN)

## Beschreibung

Es werden 9α-Hydroxy-8α-estra-1,3,5(10)-triene der Formeln Ia bis Id 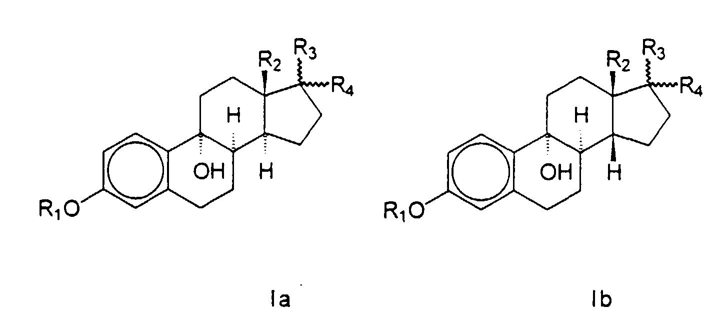 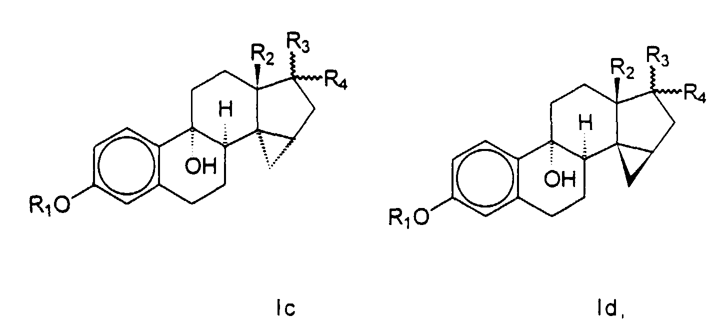 in denen R₁ ein Wasserstoffatom oder eine niedere Alkylgruppe mit 1 bis 4 Kohlenstoffatomen ist, R₂ eine Methylgruppe oder eine Ethylgruppe darstellt und R₃ und R₄ jeweils ein Wasserstoffatom, eine Hydroxygruppe, eine temporär geschützte Hydroxygruppe oder zusammen eine Oxogruppe sind, beschrieben.

Bevorzugte Verbindungen sind dabei:
3-Methoxy-8α-estra-1,3,5(10)-trien-9α,17β-diol
9α-Hydroxy-3-methoxy-8α-estra-1,3,5(10)-trien -17β-yl-acetat
9α-Hydroxy-3-methoxy-8α-estra-1,3,5(10)-trien-17-on
3-Methoxy-8α-estra-1,3,5(10)-trien-9α,17α-diol
9α-Hydroxy-3-methoxy-8α-estra-1,3,5(10)-trien-17α-yl-acetat
3-Methoxy-18a-homo-8α-estra-1,3,5(10)-trien-9α,17β-diol
9α-Hydroxy-3-methoxy-18a-homo-8α-estra-1,3,5(10)-trien-17β-yl-acetat
9α-Hydroxy-3-methoxy-18a-homo-8α-estra-1,3,5(10)-trien-17-on
8α-Estra-1,3,5(10)-trien-3,9α,17β-triol
9α-Hydroxy-8α-estra-1,3,5(10)-trien-17-on
8α-Estra-1,3,5(10)-trien-3,9α,17α-triol
14α,15α-Methylen-8α-estra-1,3,5(10)-trien-3,9α,17β-triol.

Es wird auch ein Herstellungsverfahren der erfindungsgemäßen Verbindungen la bis Id beschrieben.

Die erfindungsgemäßen Verbindungen - 9α-Hydroxy-8α-estra-1,3,5(10)-triene der Formeln la bis Id eignen sich als Steroidzwischenprodukte zur Synthese von Estra-1,3,5(10),9(11)-tetraenen der allgemeinen Formel III 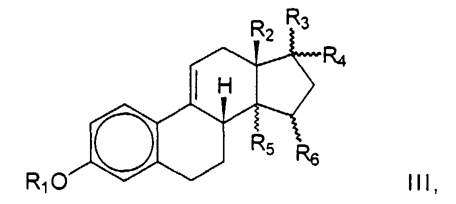 in der R₁ ein Wasserstoffatom oder eine nieder Alkylgruppe mit 1 bis 4 Kohlenstoffatomen ist, R₂ eine Methylgruppe oder eine Ethylgruppe darstellt, R₃ und R₄ jeweils ein Wasserstoffatom, eine Hydroxygruppe, eine temporär geschützte Hydroxygruppe oder zusammen eine Oxogruppe sind und R₅ und R₆ jeweils ein Wasserstoffatom oder zusammen eine Methylengruppe bedeuten.

Die Estra-1,3,5(10),9(11)-tetraene, bei welchen R₁ eine Methylgruppe, R₂ eine Ethylgruppe, R₃ eine β-Hydroxygruppe, R₄, R₅ und R₆ jeweils ein α-Wasserstoffatom bedeuten, werden nach Tetrahedron 50: 10709-10720 (1994) für die Synthese des hochwirksamen Progestagens Desogestrel verwendet.
Weiterhin sind die Estra-1,3,5(10),9(11)-tetraene für die Synthese von Steroidverbindungen geeignet, die der Estran- oder 18-Homo-estran-Reihe angehören und in Position 11 des Steroidgerüstes eine Alkylidengruppe enthalten. Verbindungen dieses Typs besitzen nach DE-PS 2 361 120 sehr wertvolle anabolische, androgene, estrogene, progestative, antiandrogene, antiestrogene, ovulationshemmende und gonadenhemmende Eigenschaften.
Ferner können nach J.Chem.Soc., Perk.Trans.1: 2595-2597 (1984) die Estra-1,3,5(10),9(11)-tetraene, in der R₁ ein Wasserstoffatom, R2 eine Methylgruppe, R₃ eine β-Hydroxygruppe, R₄ ein α-Wasserstoffatom, R₅ und R₆ ein Wasserstoffatom bedeuten, zur Synthese von Haptenen für Radioimmunoassays des Estrons eingesetzt werden; diese sind den in Position 6 des Steroidgerüstes substituierten Haptenen überlegen. Auch 11β-substituierte Derivate (Ethyl-, Chlormethyl- und Vinyl-) des Estradiols, welche sich durch eine hohe Affinität zum Estrogen-Rezeptor auszeichnen, sind nach J. Org. Chem. 60: 5316-5318 (1995) gleichfalls aus den Estra-1,3,5(10),9(11)-tetraenen synthetisierbar.

Aufgabe der Erfindung ist das Auffinden neuer Substanzen als Steroidzwischenprodukte, die, verglichen mit den bisher bekannten Synthesen zur Herstellung von Estra-1,3,5(10),9(11)-tetraenen der allgemeinen Formel III eine wesentlich vorteilhaftere Herstellung dieser Verbindungen gestatten.

Weiterhin ist Aufgabe der Erfindung, ein effektives und umweltschonendes Verfahren zur Herstellung der erfindungsgemäßen Verbindungen zu entwickeln.

Estra-1,3,5(10),9(11)-tetraene sind nach Belg. Pat. 608 369 und nach J.Chem.Soc.: 5072-5094 (1963) durch Umwandlung im sauren Medium aus Estra-1,3,5(10),8-tetraenen zugänglich.
Weiterhin werden Estra-1,3,5(10),9(11)-tetraene aus Estra-1,3,5(10),8-tetraenen dadurch hergestellt, daß man Estra-1,3,5(10),8-tetraene zunächst nach Birch zu Estra-1,3,5(10)-trienen reduziert und dann eine 9(11)-Doppelbindung in das Steroidgerüst einführt.

Nach Aust.J.Chem 36: 339-360 (1983) ist dies mit Dichlordicyanobenzochinon, nach Tetrahedron 24: 6773-6776 (1968) mitttels der Adamantylcarboniumion-Methode oder nach Tetrahedron Lett.: 2191-2192 (1977) durch Verwendung photoangeregten Nitrobenzens möglich. Nach DE-PS 25 23 179 kann es dadurch erreicht werden, daß man Estra-1,3,5(10)-triene elektrochemisch, nach Tetrahedron Lett.: 2917-2918 (1968) mit Sauerstoff in Gegenwart von Tris(triphenylphosphin)-rhodium-(I)-chlorid oder nach J.Chem.Res.(S): 28-29 (1992); J.Org.Chem 57: 2182-2184 (1992) mit Dimethyldioxiran oxidiert, wobei 9-Hydroxy- oder 9-Methoxyestra-1,3,5(10)-triene entstehen, aus denen durch Wasser- bzw. Methanol-Abspaltung Estra-1,3,5(10),9(11)-tetraene gebildet werden.

Nach J.Biol.Chem. 204: 509-515 (1953) ist die Umwandlung von Estra-1,3,5(10),8-tetraenen zu Estra-1,3,5(10),9(11)-tetraenen unter dem Einfluß von Säuren eine Gleichgewichtsreaktion. Diese Isomerisierung verläuft nur dann mit hohen Ausbeuten, wenn der D-Ring der Estra-1,3,5(10),8-tetraene stark eingeebnet ist, wodurch die praktische Verwertbarkeit des Verfahrens sehr stark einschränkt ist. Estra-1,3,5(10),8-tetraene mit einem Cyclopentanring als D-Ring und einer 17β-Hydroxy- oder 17-Oxogruppe führen nach Bull.Soc.Chim.Fr. II: 119-125, 126-129 (1978) zu Gemischen aus Estra-1,3,5(10),8-tetraenen und Estra-1,3,5(10),9(11)-tetraenen, die nur mit hohem Aufwand getrennt werden können, da sich die beiden Doppelbindungsisomeren hinsichtlich ihrer Löslichkeiten und Polaritäten nur wenig voneinander unterscheiden.

Bei der Reduktion von Estra-1,3,5(10),8-tetraenen nach Birch zu Estra-1,3,5(10)-trienen und der nachfolgenden Einführung der 9(11)-Doppelbindung wird durch die Birch-Reduktion eine zusätzliche Verfahrensstufe benötigt.
Weiterhin werden auch Substanzen als Hilfsstoffe benötigt, wie Tris(triphenylphosphin)rhodium(l)-chlorid und Dichlordicyanobenzochinon, die toxisch und umweltgefährdend sind. Die Verwendung von Dichlordicyanobenzochinon beispielsweise führt zu schwerwiegenden Problemen bei der Aufarbeitung der Reaktionsansätze, da es umfangreicher chromatographischer oder extraktiver Methoden bedarf, um das bei der Reaktion anfallende Dichlordicyanohydrochinon von den steroidalen Zielprodukten abzutrennen. Sowohl Dichlordicyanobenzochinon als auch das entsprechende Hydrochinonderivat stellen toxische, die Umwelt belastende Substanzen dar, für deren Handhabung umfangreiche Schutzvorkehrungen getroffen und die entsorgt bzw. wiedergewonnen werden müssen.

Estra-1,3,5(10),8-tetraene, aus welchen die erfindungsgemäßen Verbindungen - 9α-Hydroxy-8α-estra-1,3,5(10)-triene der Formeln Ia bis Id - hergestellt werden, fallen im Rahmen der industriellen Steroidtotalsynthese in großen Mengen an.

Erfindungsgemäß werden diese Estra-1,3,5(10),8-tetraene der Formeln IIa bis IId 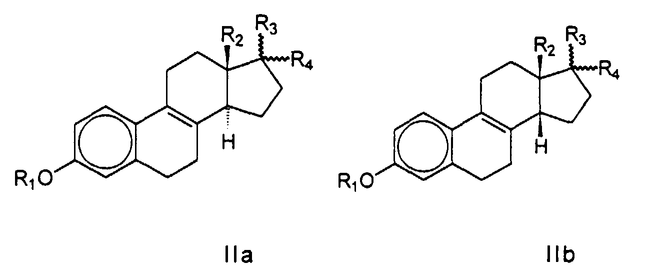 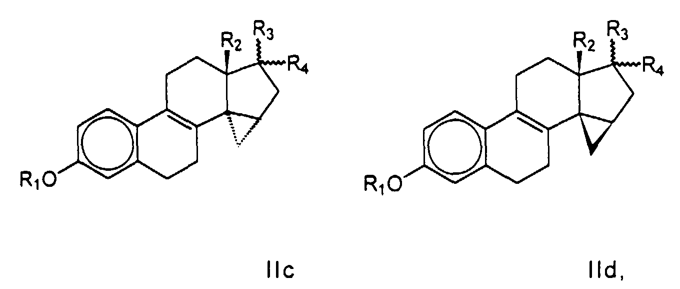 in denen R₁ ein Wasserstoffatom oder eine niedere Alkylgruppe mit 1 bis 4 Kohlenstoffatomen ist, R₂ eine Methylgruppe oder eine Ethylgruppe darstellt und R₃ und R₄ jeweils ein Wasserstoffatom, eine Hydroxygruppe, eine temporär geschützte Hydroxygruppe oder zusammen eine Oxogruppe sind,
zu den entsprechenden Organoboranen hydroboriert und diese anschließend mit alkalischem Wasserstoffperoxid zu den erfindungsgemäßen Verbindungen - 9α-Hydroxy-8α-estra-1,3,5(10)-triene der Formeln Ia bis Id 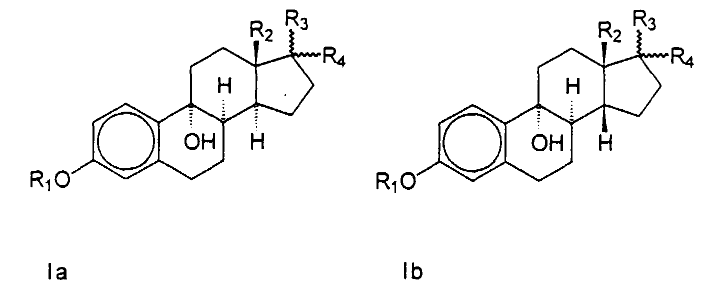 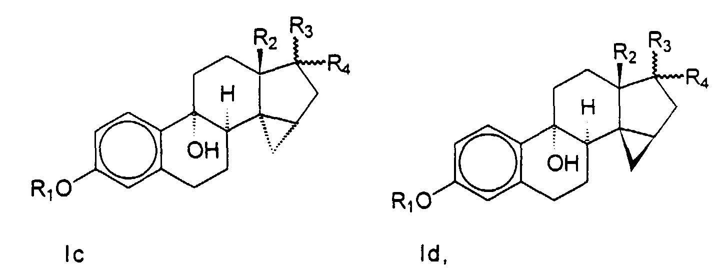 in denen R₁ ein Wasserstoffatom oder eine niedere Alkylgruppe mit 1 bis 4 Kohlenstoffatomen ist, R₂ eine Methylgruppe oder eine Ethylgruppe darstellt und R₃ und R₄ jeweils ein Wasserstoffatom, eine Hydroxygruppe, eine temporär geschützte Hydroxygruppe oder zusammen eine Oxogruppe sind,
oxydiert, wobei eine Temperatur niedriger +40 °C für einen optimalen Reaktionsablauf vorteilhaft ist.

Es wurde bisher die Hydroborierung einer 8(9)-Doppelbindung, die sich in einem Steroid mit einem aromatischen A-Ring befindet, nicht beschrieben.

Überraschenderweise wurde weiterhin gefunden, daß durch Wasserabspaltung in Gegenwart starker Lewissäuren, wie Bortribromid, Aluminiumchlorid, Aluminiumbromid, Phosphorpentachlorid, Phosphorpentabromid, Antimonpentafluorid, Antimonpentachlorid, Titan-(IV)-chlorid, Zirconium-(IV)-chlorid, Eisen(III)-chlorid, Bortrichlorid, Siliciumtetrachlorid, Siliciumtetrabromid, Niob-(V)-chlorid oder Tantal-(V)-chlorid aus den erfindungsgemäßen Verbindungen - 9α-Hydroxy-8α-estra-1,3,5(10)-triene der Formeln Ia bis Id - die Estra-1,3,5(10),9(11)-tetraenen der allgemeinen Formel III hergestellt werden können.

Dabei können der sterische Verlauf der Wasserabspaltung und die Höhe der Ausbeute an der Verbindung der allgemeinen Formel III durch die Art des verwendeten Lösungsmittels, wie beispielsweise Halogenkohlenwasserstoffe, aromatische Kohlenwasserstoffe oder Gemische derselben und durch die Reaktionstemperatur, wie -50 °C bis +20 °C, beeinflusst werden.

Die Dehydratisierung von 9-Hydroxy-estra-1,3,5(10)-trien - Steroiden kann prinzipiell zu entsprechenden 8,9-oder 9,11-Didehydro-Verbindungen oder Gemischen derselben führen, wobei die Wasserabspaltung unter Ausbildung einer 9(11)-Doppelbindung so erfolgt, daß die Konfiguration des Kohlenstoffatoms 8 unberührt bleibt.
Es wurde überraschenderweise gefunden, daß die Dehydratisierung der erfindungsgemäßen 9α-Hydroxy-8α-estra-1,3,5(10)-triene der allgemeinen Formel la bis Id mit den genannten starken Lewissäuren unter obigen Bedingungen zur Ausbildung einer 9(11)-Doppelbindung und zugleich zur Konfigurationsumkehr am Kohlenstoffatom 8, d.h. zu den Estra-1,3,5(10),9(11)-tetraenen der allgemeinen Formel III führt.

Ein Vorteil des Verfahrens zur Herstellung der Estra-1,3,5(10),9(11)-tetraene aus den erfindungsgemäßen Verbindungen - 9α-Hydroxy-8α-estra-1,3,5(10)-triene der Formeln 1a bis 1d - besteht darin, daß der regio- und stereoselektive Verlauf der Dehydratisierung von 9α-Hydroxy-8α-Steroiden der allgemeinen Formeln la bis Id, der, neben der Einführung einer 9(11)-Doppelbindung in das Molekül, die Ausbildung einer B/C-Ringverknüpfung mit einem 8β-ständigen Wasserstoff zur Folge hat, die Herstellung "natürlich" konfigurierter Zielverbindungen der allgemeinen Formel III erlaubt, aus welchen therapeutisch relevante 11-substituierte Steroidhormone zugänglich sind.
Weiterhin ist das Verfahren auch zur Herstellung von Equilenin und 8α-Estra-1,3,5(10), 9(11)-tetraenen geeignet.

Das erfindungsgemäße Verfahren wird anhand eines ausgewählten Beispiels im Vergleich zu einem bekannten Verfahren - nach Tetrahedron 50: 10709 - 10720, 1994 - durch das nachfolgende Formelschema verdeutlicht.

Es ist ersichtlich, daß durch die Verwendung der erfindungsgemäßen Verbindungen der allgemeinen Formel la bis Id eine Verkürzung des Verfahrensweges zur Herstellung von Estra-1,3,5(10),9(11)-tetraenen der allgemeinen Formel III realisiert wird.

Man vermeidet den Einsatz toxischer und umweltgefährdender Hilfsstoffe, Prozesse zum Recycling dieser Stoffe und deren Entsorgung. 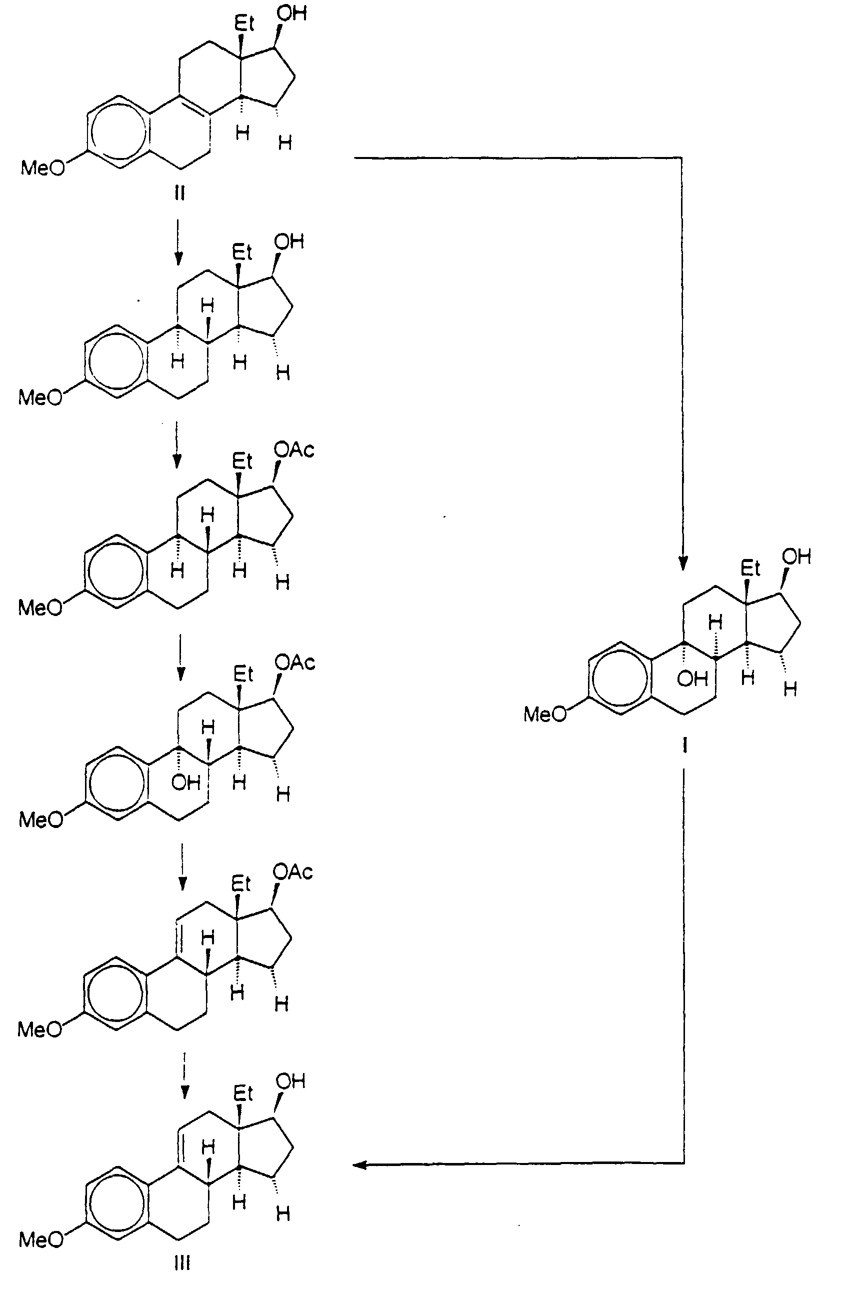 Die erfindungsgemäßen Verbindungen und ihre Herstellung werden nachfolgend beispielhaft, aber nicht einschränkend, beschrieben.

### Beispiel 1

### 3-Methoxy-8α-estra-1,3,5(10)-trien-9α,17β-diol

10 g 3-Methoxy-estra-1,3,5(10),8(9)-tetraen-17β-ol werden unter Argon in 25 ml 1,2-Dimethoxyethan suspendiert. Nach Zugabe von 28,1 g Boran-Dimethylsulfid-Komplex wird 2,5 Stunden bei +23 °C gerührt. Anschließend setzt man unter Kühlung vorsichtig 30 ml Wasser zu. Nach beendeter Gasentwicklung werden 35 ml 3N Natronlauge hinzu gegeben. Unter Kühlung des Reaktionsgemisches tropft man 35 ml 30%iges Wasserstoffperoxid zu und rührt nach beendeter Zugabe eine Stunde bei Raumtemperatur. Abschließend wird mit Wasser verdünnt und mit Ethylacetat extrahiert.
Reinigung des Rohproduktes an Kieselgel ergibt 3-Methoxy-8α-estra-1,3,5(10)-trien-9α,17β-diol als farblosen Schaum.
[α]_{D}: -6° (CHCl₃, c=1,0); ¹H-NMR (CDCl₃, 300 MHz) δ [ppm]: 0,85 (s, 3H), 3,73 (t, 1H), 3,78 (s, 3H), 6,57 (d, 1H), 6,79 (dd, 1H), 7,48 (d, 1H).

### Beispiel 2

### 3-Methoxy-estra-1,3,5(10),9(11)-tetraen-17β-diol

3 g 3-Methoxy-8α-estra-1,3,5(10)-trien-9α,17β-diol, hergestellt nach Beispiel 1, werden in 200 ml Dichlormethan gelöst. Die Lösung kühlt man unter Argonschutz auf -20 °C. Daraufhin setzt man 1,13 ml Siliciumtetrachlorid zu und rührt 10 Minuten bei -20 °C. Nach Ablauf dieser Zeit werden 500 ml gesättigte wäßrige Natriumhydrogencarbonatlösung zugesetzt.

Extraktion mit Ethylacetat und Umkristallisation ergeben 3-Methoxy-estra-1,3,5(10),9(11)-tetraen-17β-diol vom Schmelzpunkt 98 bis 100°C (Methanol).

### Beispiel 3

### 8α-Estra-1,3,5(10)-trien-3,9α,17β-triol

390 mg Estra-1,3,5(10),8(9)-tetraen-3,17β-diol werden in 1 ml Tetrahydrofuran unter Argon vorgelegt. Man gibt 15,8 ml 1M Boran-Tetrahydrofuran-Komplex-Lösung in Tetrahydrofuran zu und rührt 2 Stunden bei Raumtemperatur. Anschließend werden unter Kühlung 5 ml Wasser zugetropft. Nach beendeter Gasentwicklung werden 5 ml 3N Natronlauge zugesetzt und unter Kühlung langsam 5 ml 30%iges Wasserstoffperoxid zugetropft. Man rührt nach beendeter Zugabe eine Stunde bei Raumtemperatur. Abschließend wird mit Wasser verdünnt, neutralisiert und mit Ethylacetat extrahiert.
Man erhält 8α-Estra-1,3,5(10)-trien-3,9α,17β-triol vom Schmelzpunkt 180 bis 182,5°C (Ethylacetat).
[α]_{D}: -6° (Dioxan, c=1,0); ¹H-NMR (DMSO-d₆, 300 MHz) δ [ppm]: 0,71 (s, 3H), 4,38 (s, 1H), 4,45 (d, 1H), 6,36 (d, 1H), 6,55 (dd, 1H), 7,28 (d, 1H), 9,06 (s, 1H).

### Beispiel 4

### Estra-1,3,5(10),9(11)-tetraen-3,17β-diol

200 mg 8α-Estra-1,3,5(10)-trien-9α,17β-diol, hergestellt nach Beispiel 3, werden in 10 ml 1,2-Dichlorethan gelöst. Die Lösung kühlt man unter Argonschutz auf -20 °C. Daraufhin setzt man 0,08 ml Siliciumtetrachlorid zu und rührt 10 Minuten bei -20 °C. Nach Ablauf dieser Zeit werden 50 ml gesättigte wäßrige Natriumhydrogencarbonatlösung zugesetzt.
Extraktion mit Ethylacetat und Umkristallisation ergeben Estra-1,3,5(10),9(11)-tetraen-3,17β-diol vom Schmelzpunkt 185 bis 188°C (Methanol).

### Beispiel 5

### 3-Methoxy-18a-homo-8α-estra-1,3,5(10)-trien-9α,17β-diol

1 g 3-Methoxy-18a-homo-estra-1,3,5(10),8(9)-tetraen-17β-ol sowie 10 ml 1,2-Dimethoxyethan werden unter Argonschutz vorgelegt und mit 2,45 g Boran-Dimethylsulfid-Komplex versetzt. Man rührt vier Stunden bei 35 °C. Anschließend wird unter Kühlung eine Mischung aus 5 ml Wasser und 5 ml Methanol vorsichtig zugetropft. Nach beendeter Gasentwicklung werden 10 ml 3N Natronlauge zugefügt und unter Kühlung 10 ml 30%iges Wasserstoffperoxid langsam zugetropft. Ist die Zugabe beendet, rührt man noch 30 Minuten bei Raumtemperatur, verdünnt nachfolgend mit Wasser und extrahiert mit Ethylacetat.
Reinigung der Rohproduktes an Kieselgel ergibt 3-Methoxy-18a-homo-8α-estra-1,3,5(10)-trien-9α,17β-diol vom Schmelzpunkt 137 bis 140°C (Methyl-tert.butylether).
[α]_{D}: -13° (CHCl₃, c=1,0); ¹H-NMR (CDCl₃, 300 MHz) δ [ppm]: 0,97 (t, 3H), 3,78 (s, 3H), 3,85 (t, 1H), 6,56 (d, 1H), 6,79 (dd, 1H), 7,48 (d, 1H).

### Beispiel 6

### 3-Methoxy-18a-homo-estra-1,3,5(10),9(11)-tetraen-17β-ol

1 g 3-Methoxy-18a-homo-8α-estra-1,3,5(10)-trien-9α,17β-diol, hergestellt nach Beispiel 5, wird in 100 ml Methylenchlorid gelöst und die Lösung unter Argon auf -20 °C gekühlt. Daraufhin setzt man 3,36 ml einer 1M Bortrichloridlösung in Methylenchlorid zu und rührt 10 Minuten bei -20 °C. Nach Ablauf dieser Zeit werden 300 ml gesättigte wäßrige Natriumhydrogencarbonatlösung zugesetzt.
Extraktion mit Ethylacetat und Umkristallisation ergeben 3-Methoxy-18a-homo-estra-1,3,5(10),9(11)-tetraen-17β-diol vom Schmelzpunkt 50 bis 53°C (Methanol).

## Patentansprüche

1. 9α-Hydroxy-8α-estra-1,3,5(10)-triene der Formeln Ia bis Id 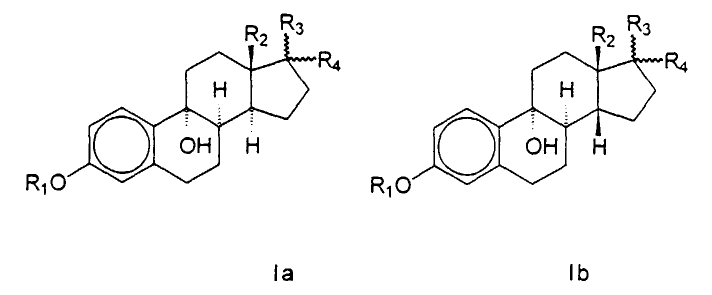 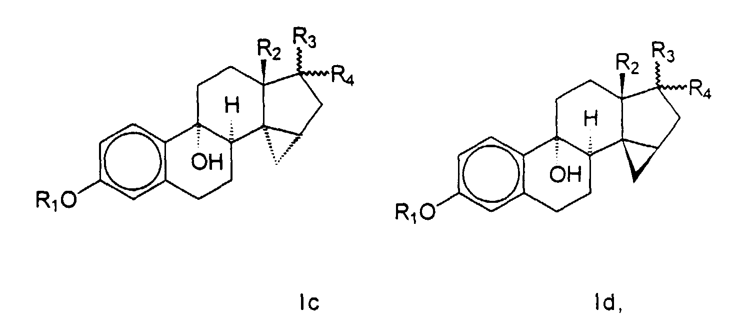 in denen R₁ ein Wasserstoffatom oder eine niedere Alkylgruppe mit 1 bis 4 Kohlenstoffatomen ist, R₂ eine Methylgruppe oder eine Ethylgruppe darstellt und R₃ und R₄ jeweils ein Wasserstoffatom, eine Hydroxygruppe, eine temporär geschützte Hydroxygruppe oder zusammen eine Oxogruppe sind.

2. Verfahren zur Herstellung von 9α-Hydroxy-8α-estra-1,3,5(10)-trienen der Formeln la bis Id nach Anspruch 1,
dadurch gekennzeichnet, daß man
Estra-1,3,5(10),8-tetraene der Formeln lla bis lld 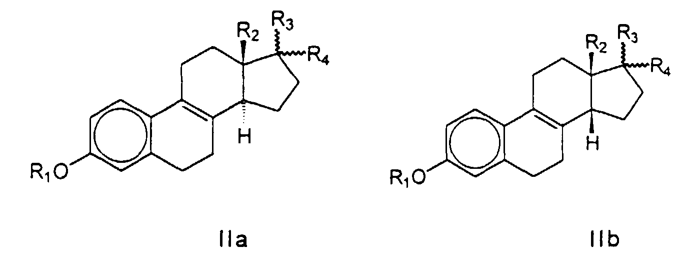 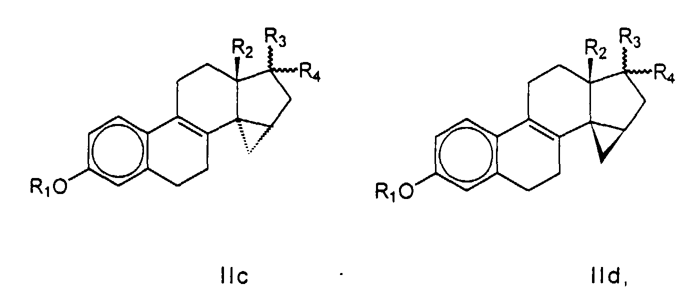 in denen R₁ ein Wasserstoffatom oder eine niedere Alkylgruppe mit 1 bis 4 Kohlenstoffatomen ist, R₂ eine Methylgruppe oder eine Ethylgruppe darstellt und R₃ und R₄ jeweils ein Wasserstoffatom, eine Hydroxygruppe, eine temporär geschützte Hydroxygruppe oder zusammen eine Oxogruppe sind,
zu den entsprechenden Organoboranen hydroboriert und diese anschließend mit alkalischem Wasserstoffperoxid oxydiert.

3. Verfahren nach Anspruch 2,
dadurch gekennzeichnet, daß man
bei maximal + 40 °C hydroboriert.

## Claims

1. 9α-Hydroxy-8α-oestra-1,3,5(10)-trienes of formulae Ia to Id: 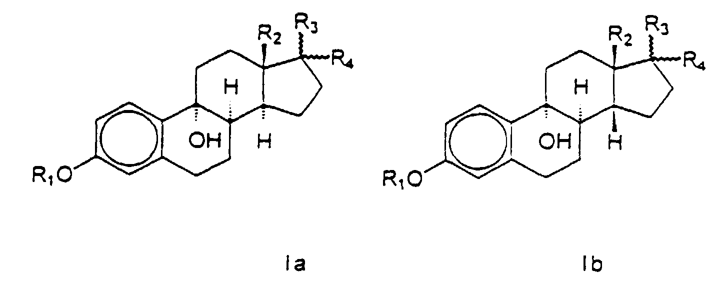 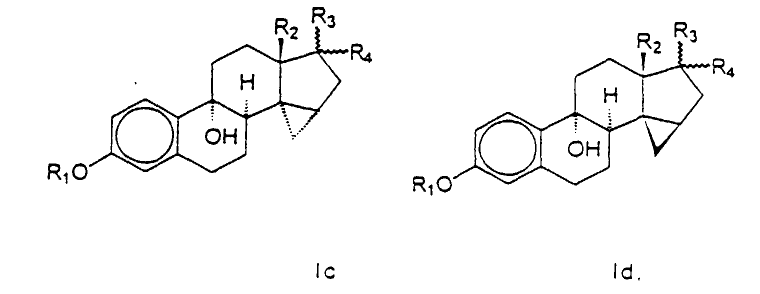 in which R₁ is a hydrogen atom or a lower alkyl group having 1 to 4 carbon atoms, R₂ is a methyl group or an ethyl group and R₃ and R₄ are each a hydrogen atom, a hydroxyl group or a temporarily protected hydroxyl group or together are an oxo group.

2. Process for the preparation of 9α-hydroxy-8α-oestra-1,3,5(10)-trienes of formulae Ia to Id according to Claim 1, characterized in that oestra-1,3,5(10),8-tetraenes of formulae IIa to IId: 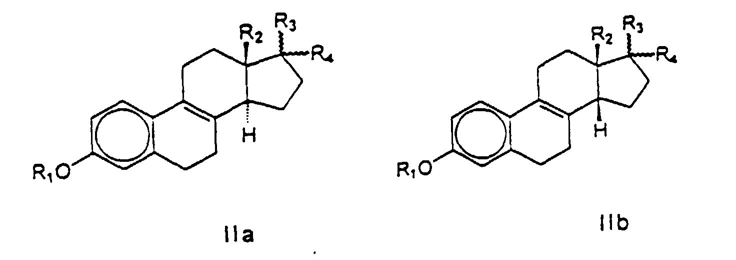 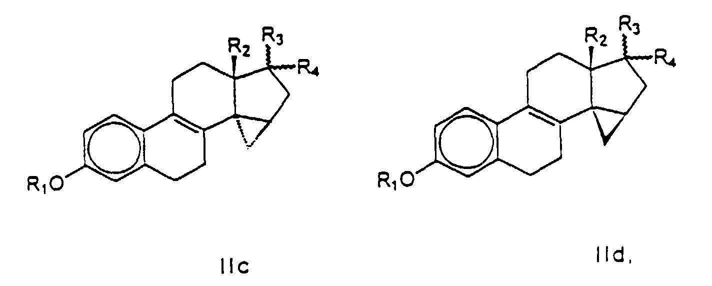 in which R₁ is a hydrogen atom or a lower alkyl group having 1 to 4 carbon atoms, R₂ is a methyl group or an ethyl group and R₃ and R₄ are each a hydrogen atom, a hydroxyl group or a temporarily protected hydroxyl group or together are an oxo group,
are hydroborated to the corresponding organoboranes, which are then oxidized with alkaline hydrogen peroxide.

3. Process according to Claim 2, characterized in that the hydroboration is carried out at a maximum of +40°C.

## Revendications

1. 9α-hydroxy-8α-oestra-1,3,5(10)-triène des formules Ia à Id 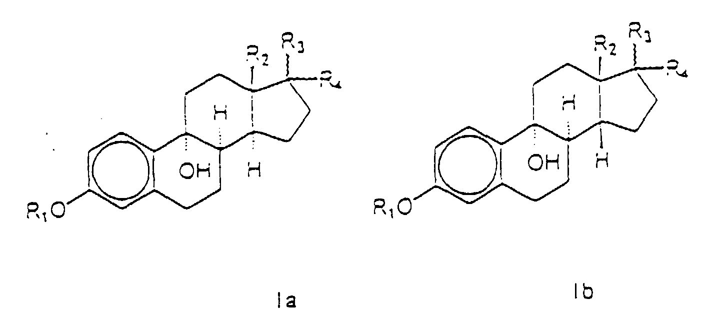 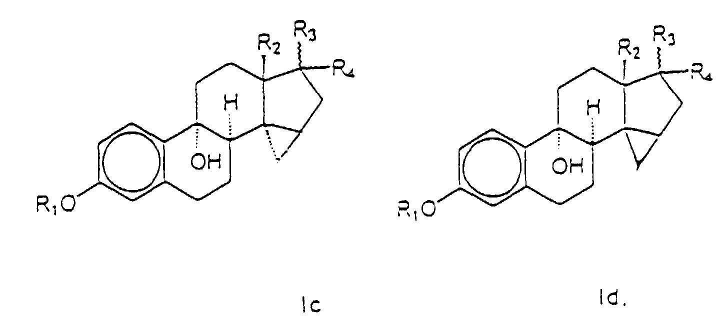 dans lesquelles R₁ est un atome d'hydrogène ou un groupe alkyle inférieur avec 1 à 4 atomes de carbone, R₂ représente un groupe méthyle ou bien un groupe éthyle et R₃ et R₄ sont chacun un atome d'hydrogène, un groupe hydroxy, un groupe hydroxy temporairement protégé ou bien ils forment ensemble un groupe oxo.

2. Procédé pour la production de 9α-hydroxy-8α-oestra-1,3,5(10)-triènes des formules la à Id selon la revendication 1,
caractérisé en ce
qu'un oestra-1,3,5(10),8-tétraène des formules IIa à IId 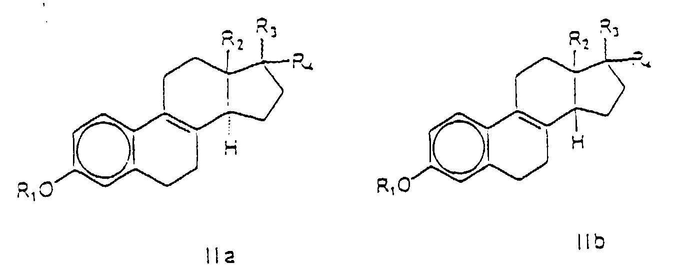 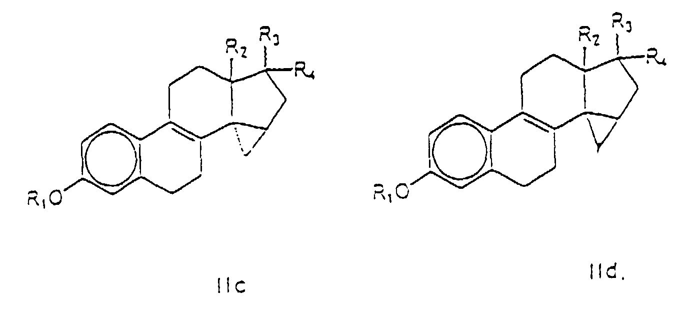 dans lesquelles R₁ est un atome d'hydrogène ou un groupe alkyle inférieur avec 1 à 4 atomes de carbone, R₂ représente un groupe méthyle ou bien un groupe éthyle et R₃ et R₄ représentent à chaque fois un atome d'hydrogène, un groupe hydroxy, un groupe hydroxy temporairement protégé ou bien ils forment ensemble un groupe oxo,
est hydroboré en organoboranes correspondants et ceux-ci sont subséquemment oxydés avec un péroxyde d'hydrogène alcalin.

3. Procédé selon la revendication 2,
caractérisé en ce
qu'on effectue l'hydroboruration au maximum à + 40°C.
